(19) Europäisches Patentamt
European Patent Office
Office européen des brevets

(11) Publication number: **0 354 622**
**A1**

(12) **EUROPEAN PATENT APPLICATION**

(21) Application number: **89202058.7**

(51) Int. Cl.⁴: **C07D 271/113 , A01N 43/82**

(22) Date of filing: **08.08.89**

(30) Priority: **11.08.88 GB 8819120**
**09.03.89 GB 8905377**

(43) Date of publication of application:
**14.02.90 Bulletin 90/07**

(84) Designated Contracting States:
**BE CH DE ES FR GB IT LI NL**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Clark, Michael Thomas**
**Torcross Wises Lane**
**Borden Sittingbourne Kent(GB)**
Inventor: **Gilmore, Ian James**
**32 Waterloo Road**
**Sittingbourne Kent(GB)**

(74) Representative: **Bennett, David Arthur Horder et al**
**4, York Road**
**London SE1 7NA(GB)**

(54) **Oxadiazolone herbicides.**

(57) The invention provides oxadiazolone compounds having the general formula I:

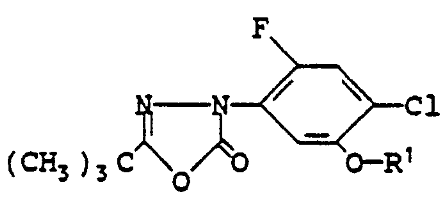

(I)

wherein $R^1$ represents an alkoxycarbonylalkyl, methods for their preparation and their use as herbicides.

EP 0 354 622 A1

## OXADIAZOLONE HERBICIDES

This invention relates to certain oxadiazolone compounds and their preparation, herbicidal compositions containing these compounds, and their use in combating undesired plant growth.

It is known, from UK Patent Nos. 1 063 799, 1 110 500 and 1 353 839 for example, that oxadiazolone compounds having the general formula:

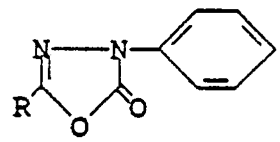

wherein R represents an alkyl group, and corresponding compounds in which the phenyl ring is substituted by one or more moieties independently selected from halogen atoms, alkyl and alkoxy groups and nitro groups, are active as herbicides. UK Patent No. 1,110,500 specifically provides such compounds having the general formula

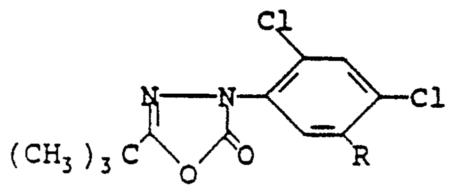

wherein R represents an alkoxy group of 1 to 4 carbon atoms.

Further, European Patent Application publication No. 274 249 discloses oxadiazolone compounds having the general formula:

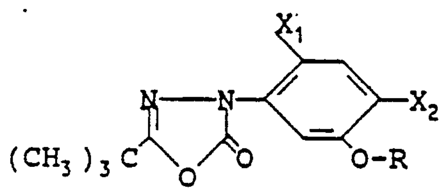

wherein $X_1$ is a fluorine atom, $X_2$ is a chlorine atom, and R is a $C_{1-6}$ alkyl group or a $C_{3-4}$ alkenyl or alkynyl group, and their use as herbicides. The specification of European Patent Application publication No. 274 249 is particularly concerned with the compound 5-t-butyl-3-(4-chloro-2-fluoro-5-propargyloxy-phenyl)-1,3,4-oxadiazol-2-one, which may be given the alternative name 5-tert-butyl-3-(2-fluoro-4-chloro-5-(2-propynyloxy)phenyl)-1,3,4-oxadiazol-2(3H)-one, the claims of this application being directed solely to this compound, a process for its preparation and its use as a herbicide.

However, a certain novel group of herbicidally effective compounds has now unexpectedly been found to possess a significantly higher degree of selectivity than analogous previously disclosed compounds, particularly in one or more of soya, wheat and barley.

Accordingly, the present invention provides oxadiazolone compounds having the general formula I:

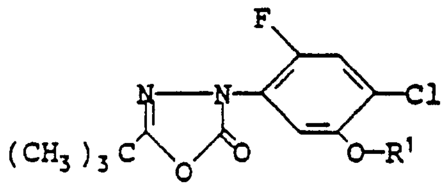

(I)

wherein $R^1$ represents an alkoxycarbonylalkyl group.

Alkyl and alkoxy groups preferably have 1 to 12 carbon atoms, especially 1 to 6 carbon atoms. An alkyl or alkoxy group may be straight chain or branched.

Preferably $R^1$ represents a ($C_{1-4}$ alkoxy)carbonyl($C_{1-4}$alkyl) group, more preferably a ($C_{1-2}$) alkoxycarbonyl($C_{1-2}$alkyl) group. Especially preferred compounds are those in which $R^1$ represents ethoxycarbonylmethyl and 1-(ethoxycarbonyl)ethyl.

It will be appreciated that when the groups represented by $R^1$ include an asymmetric carbon atom, the compounds of formula I will exist in sterioisomeric forms. The scope of the present invention includes all individual isomeric forms of the compounds of formula I and mixtures of isomers and further includes herbicidal compositions containing the active ingredient in such isomeric forms and mixtures.

The compounds of formula I in which $R^1$ is replaced by a hydrogen atom are themselves effective as herbicides. However, they are also useful as intermediates in the preparation of compounds of formula I.

Accordingly, the present invention also provides a process for the preparation of an oxadiazolone compound of formula I as defined above which comprises reacting the compound having the formula II:

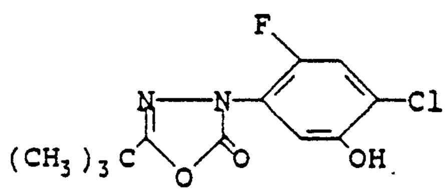

(II)

with a compound of formula $R^1$-Y wherein $R^1$ is as hereinbefore defined and Y represents a leaving group in a solvent in the presence of a base.

Suitable leaving groups represented by Y for the preparation of compounds of formula I include halogen atoms, for example bromine and iodine, and sulfonic ester groups, especially tosylate and mesylate. Suitable solvents are, for example, acetone and acetonitrile. Bases suitable for use in this reaction include alkali metal carbonates, for example the carbonates of lithium, potassium and sodium, alkali metal hydroxides and alkali metal alkoxides. The reaction may conveniently be effected at reflux temperature.

For such preparations involving compounds of formula $R^1$-Y the reaction can be allowed to proceed in the presence of a complexing agent, for example a crown ether. Potassium carbonate is a very suitable base, in which case, the complexing agent is preferably 1,4,7,10,13,16-hexaoxycyclooctadecane (18-Crown-6). Alternative combinations of complexing agents and bases that are suitable for use in this reaction include lithium carbonate together with 1,4,7,10-tetraoxacyclododecane (12-Crown-4), and sodium carbonate together with 1,4,7,10,13-pentaoxacyclopentadecane (15-Crown-5).

The compound of formula II may be prepared by treating compounds having the general formula III:

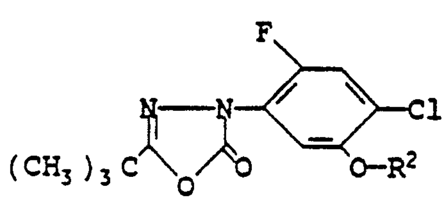

(III)

wherein $R^2$ represents an alkyl group, with a Lewis acid in a suitable organic solvent, for example methylene chloride. Suitable Lewis acids include boron tribromide and aluminium trichloride. Alternative solvents that may be used are, for example, chloroform and carbon tetrachloride.

The reaction may be effected by mixing the reagents together at a temperature in the range of 0 °C to

40 ° C, typically ambient temperature (about 20 ° C).

This reaction may also be carried out using a mineral acid, for example hydrochloric acid or hydrobromic acid, in a suitable solvent such as acetic acid. As a further alternative, the reaction may be performed under neutral conditions, for example by treating the aforementioned compounds of formula III with iodotrimethylsilane in a suitable solvent, such as acetonitrile.

Compounds of formula III may be prepared by diazotising compounds having the general formula IV

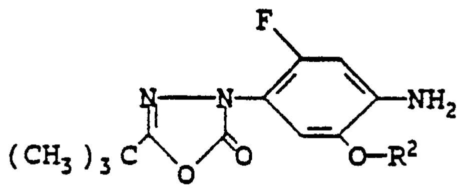

(IV)

wherein $R^2$ is as hereinbefore defined, to give the corresponding diazonium salt, followed by converting the diazonium salt to the compound of formula III as hereinbefore defined.

Diazotisation of the compound of formula IV to give the corresponding diazonium salt may be carried out according to established procedures by treating the compound of formula IV with a nitrosating agent, for example sodium nitrite. The diazonium salt may conveniently be prepared by reacting the compound of formula IV with sodium nitrite and an inorganic acid, preferably hydrochloric acid, in aqueous medium. Alternatively, the diazonium salt may be prepared, for example, by reaction of the compound of formula IV with an aqueous solution of nitrous acid and hydrochloric acid, with an aqueous solution of nitric acid and dinitrogen trioxide, with a solution of nitric acid and sodium thiosulphite, or with organic nitrite compounds, for example pentylnitrite in an organic solvent, for example tetrahydrofuran.

The diazotisation may conveniently be carried out at a temperature ranging from the freezing point of the reaction mixture to 5 ° C, preferably at a temperature of about 0 ° C.

Conversion of the diazonium salt to the compound of formula III may be carried out by procedures well known to the person skilled in the art. Preferably, the diazonium salt is treated with an aqueous solution of cuprous chloride and hydrochloric acid. Alternatively, the cuprous salt may be replaced by the equivalent cupric salt or by copper powder. These reactions are performed by adding a solution of the diazonium salt at a temperature at or below 5 ° C, preferably about 0 ° C, to an aqueous solution of the selected copper based reagent in the appropriate acid at a similarly low temperature and then heating the reaction mixture to a temperature in the range of 20 ° C to 80 ° C, preferably about 60 ° C.

Compounds of formula IV may be prepared by reacting the corresponding compound of the general formula V:

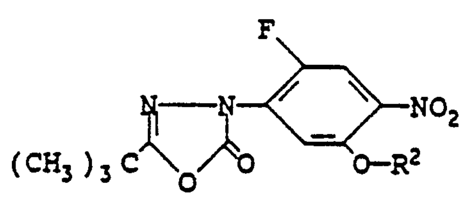

(V)

wherein $R^2$ is as hereinbefore defined, with a reducing agent. For example, a compound of formula V may conveniently be dissolved in an organic solvent, such as tetrahydrofuran, and treated with a suitable reducing agent, such as an aqueous solution of sodium hypophosphite. Preferably, the reaction is performed in the presence of a catalyst. If sodium hypophosphite is used as reducing agent, for example, the catalyst is suitably a palladium/charcoal catalyst. Other suitable reducing agents for this reaction include hydrazine, preferably used together with platinum (IV) oxide (Adam's catalyst), and iron or zinc in acetic acid. Alternative organic solvents that may be used are, for example, methanol, ethanol, isopropanol and dioxan.

Compounds of formula V may be prepared by reacting a corresponding hydrazide of the general formula VI:

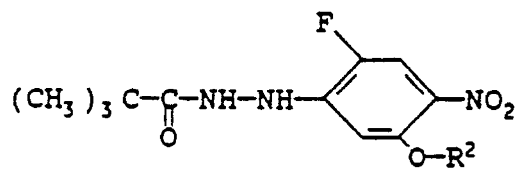

(VI)

wherein $R^2$ is as hereinbefore defined, in a suitable organic solvent, for example toluene, with phosgene, conveniently at the reflux temperature of the reaction mixture. Possible alternatives to phosgene as a reagent include trichloromethyl chloroformate (diphosgene) and other chloroformate esters, for example, methyl chloroformate and ethyl chloroformate. Other suitable solvents for this reaction include benzene, chlorobenzene and xylene.

The hydrazides of formula VI may be prepared by reacting a corresponding hydrazide of the general formula VII:

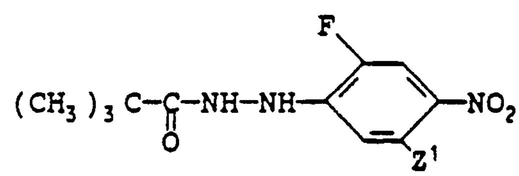

(VII)

wherein $Z^1$ represents a halogen atom, preferably a fluorine or chlorine atom, with an alkali metal alkoxide of formula $R^2$-OM wherein $R^2$ is as defined above and M is an alkali metal, preferably sodium. For example, the hydrazide of formula VII is dissolved in an organic solvent, for example tetrahydrofuran, and the solution is added to a solution of sodium methoxide in methanol, at a temperature in the range of 0 °C to 5 °C.

Hydrazides of formula VII may be obtained by reaction of a phenyl hydrazine of the general formula VIII:

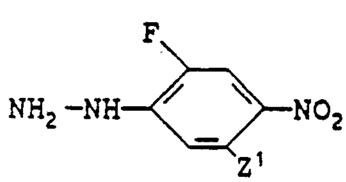

(VIII)

wherein $Z^1$ is as defined above, with 2,2-dimethylpropanoyl chloride in a suitable organic solvent, for example methylene chloride, and in the presence of a base, for example triethylamine. Alternative bases suitable for this reaction include piperidine, pyridine and inorganic bases, for example sodium bicarbonate. Suitable solvents that may alternatively be used in the reaction include chloroform, carbon tetrachloride, tetrahydrofuran and dioxan.

The phenyl hydrazine of formula VIII may be prepared from a substituted benzene compound of the general formula IX:

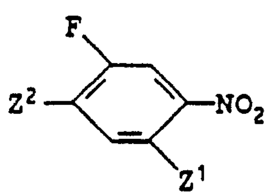

(IX)

wherein $Z^1$ is as hereinbefore defined and $Z^2$ represents a halogen atom, preferably a fluorine or chlorine atom, by reaction with hydrazine in an organic solvent, such as methanol.

Compounds of formula IX are known and are readily obtainable by procedures well known to the person skilled in the art, for example those described by Finger et al, J. Am. Chem. Soc. 73, 145, (1951).

Compounds of formula I have been found to have interesting activity as herbicides, in particular in their performance in selectively controlling weeds, especially broad leaved weeds, in crops such as soya, wheat and barley. Accordingly, the present invention also provides a herbicidal composition comprising a compound of formula I as defined above, in association with at least one carrier.

The present invention also provides the use of such a compound or composition according to this invention as a herbicide.

Further, in accordance with the present invention there is provided a method of combating undesired plant growth at a locus by treating the locus with a compound or composition according to the invention. Application to the locus may be pre-emergence or post-emergence. The dosage of the active ingredient used may be, for example, from 0.001 to 10kg/ha, preferably 0.01 to 5 kg/ha, especially 0.01 to 2 kg/ha.

A carrier in a composition according to the invention is any material with which the active ingredient is formulated to facilitate application to the locus to be treated, which may for example be a plant, seed or soil, or to facilitate storage, transport or handling. A carrier may be a solid or a liquid, including a material which is normally gaseous but which has been compressed to form a liquid, and any of the carriers normally used in formulating herbicidal compositions may be used. Preferably compositions according to the invention contain 0.5 to 95% by weight of active ingredient

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation of fatty acids or aliphatic amines or amides containing at least 12 carbon atoms in the molecule with ethylene oxide and/or propylene oxide; fatty acid esters of glycerol, sorbitol, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3-10% w of a dispersing agent and, where necessary, 0-10% w of stabiliser(s) and/or other additives such as penetrants or stickers. DUStS are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-75% w active ingredient and 0-10% w of additives such as stabilisers, surfactants, slow release modifiers and binding agents. The so-called "dry flowable powders" consist of relatively small granules having a relatively high concentration of active

ingredient. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the invention. The said emulsions may be of the water-in-oil or of the oil-in-water type, and may have a thick 'mayonnaise'-like consistency.

The composition of the invention may also contain other ingredients, for example other compounds possessing herbicidal, insecticidal or fungicidal properties.

The invention will be further understood from the following Examples, in which Example 1 relates to the preparation of the intermediate of formula II, Examples 2 and 3 relate to the preparation of compounds according to the present invention and Examples 4, 5 and 6 relate to herbicidal tests.

## Example 1

### Preparation of 5-tert-butyl-3-(2-fluoro-4-chloro-5-hydroxyphenyl)-1,3,4-oxadiazol-2(3H)-one

a) 1,2,4-Trifluoro-5-nitrobenzene (88.5g, 0.5mol) was dissolved in methanol (500ml). A solution of hydrazine (16g, 0.5mol) dissolved in methanol (100ml) was added dropwise producing a dark red colouration. The reaction mixture was allowed to stand for about 2 hours, during which the mixture became orange/red in colour, and was then heated under reflux for 30 minutes, followed by solvent evaporation. The residue was added to a mixture of a dilute aqueous sodium bicarbonate solution and methylene chloride and the resulting mixture stirred and then allowed to separate into two phases. The methylene chloride phase was separated from the mixture and the aqueous phase was extracted with methylene chloride (4 x 150ml). The methylene chloride phase and extracts were combined and washed with water (100ml), dried using sodium sulphate and the solvent evaporated. The residue, a mixture of deep red and orange crystals, was chromatographically purified on a silica column with methylene chloride as eluant. The product, 2,5-difluoro-4-nitrophenylhydrazine, was isolated from the third fraction eluted from the silica column as orange crystals in a yield of 37.8g, m.pt. 150 °C.

| | | | | |
|---|---|---|---|---|
| Analysis: | Calculated: | 38.1% C | 2.65% H | 22.2% N |
| | Found: | 37.9% C | 2.7% H | 22.3% N |

b) The product of a) (18.9g, 0.1mol) was dissolved in dry methylene chloride (140ml). Triethylamine was added (10ml, 0.1mol) and the resulting mixture was cooled to 0 °C. Pivaloyl chloride (12.3g, 0.1mol) was added dropwise with stirring and the reaction mixture was stirred at ambient temperature (20 °C) overnight. Water was added and the resulting mixture was filtered to separate bright yellow crystals. The crystals were washed with water and dried. The product, 1-(2,5-difluoro-4-nitrophenyl)-2-(2,2-dimethylpropanoyl)hydrazine, was isolated by recrystallization from a mixture of ethyl acetate and hexane as bright yellow crystals in a yield of 23.5g, m.pt 152 to 154 °C.

| | | | | |
|---|---|---|---|---|
| Analysis: | Calculated: | 48.35% C | 4.8% H | 15.4% N |
| | Found: | 47.2 % C | 5.0% H | 15.8% N |

c) The product of b) (6.4g, 0.0234mol) was dissolved in dry tetrahydrofuran (50ml) and added to a solution of sodium (1.65g, 0.072mol) in dry methanol (30ml) at a temperature of 0 °C. The resulting mixture

was heated under reflux for 3 hours and then stirred overnight at ambient temperature (20 ° C). The solvent was removed and the residue was acidified with 2N hydrochloric acid. The mixture was extracted with ethyl acetate (3x100ml) and the combined extracts washed with water (100ml) and then dried using sodium sulphate. The combined extracts were then filtered and the solvent was evaporated. The product, 1-(2-fluoro-5-methoxy-4-nitrophenyl)-2-(2,2-dimethylpropanoyl)hydrazine, was isolated as pale brown crystals, by recrystallization from ethanol (75ml), in a yield of 4.2g, m.pt. 164 to 166 ° C.

| | | | | |
|---|---|---|---|---|
| Analysis: | Calculated: | 50.5% C | 5.6% H | 14.7% N |
| | Found: | 50.0% C | 5.6% H | 14.6% N |

d) The product of c) (3.6g, 0.0126mol) was dissolved in dry toluene (40ml). A solution of 20%w/v phosgene in toluene (23.5ml, 0.049mol) was added and the resulting mixture was heated under reflux with stirring overnight. The mixture was cooled and the solvent evaporated. The residue was added to a mixture of water and trichloromethane with stirring. After allowing the mixture to separate into two phases, the organic phase was separated from the mixture and the aqueous phase was extracted with trichloromethane (3x100ml). The organic phase and extracts were combined and dried using sodium sulphate. Chromatographic purification on a silica column with trichloromethane as eluant yielded the product, 5-tert-butyl-3(2-fluoro-5-methoxy-4-nitrophenyl)-!,3,4-oxadiazol-2(3H)-one, as a pale yellow oil from the first fraction eluted from the column. The product was solidified by trituration with hexane to a white solid in a yield of 2.7g, m.pt 111 to 112 ° C.

| | | | | |
|---|---|---|---|---|
| Analysis: | Calculated: | 50.2% C | 4.5% H | 13.5% N |
| | Found: | 50.1% C | 4.5% H | 13.6% N |

e) The product of d) (11.5g, 0.037mol) was dissolved in tetrahydrofuran (300ml). 5%w/w Palladium/Charcoal (0.5g) was added to the mixture, followed by an aqueous solution of 15%w/v sodium hypophosphite (250ml) added dropwise over a period of 15 minutes. The resulting mixture was stirred for 45 minutes and was then placed in a rotary evaporator for organic solvent removal. The remaining aqueous mixture was combined with ethyl acetate and the resulting mixture filtered. The filtrate was washed and extracted with ethyl acetate (3x100ml). The washings and extracts were combined, washed with water and dried using sodium sulphate. The solvent was evaporated to leave an orange oil as residue. The residue was triturated with hexane to obtain the product, 5-tert-butyl-3-(2-fluoro-5-methoxy-4-phenylamino)-1,3,4-oxadiazol-2(3H)-one, as pale orange crystals in a yield of 10.4g, m.pt. 139 ° C.

| | | | | |
|---|---|---|---|---|
| Analysis: | Calculated: | 55.2% C | 5.7% H | 14.95% N |
| | Found: | 55.3% C | 5.9% H | 14.0% N |

f) The product of e) (7.35g, 0.026mol) was suspended in a mixture of concentrated hydrochloric acid (38ml) and water (38ml). The resulting mixture was cooled to 0 ° C and a solution of sodium nitrite (2.3g, 0.033mol) in water (5ml) was added dropwise. After the addition had been completed, the reaction mixture was stirred for 30 minutes and then added to a solution of copper (I) chloride (5.25g, 0.053mol) in concentrated hydrochloric acid (38ml) held at a temperature of 0 ° C. The resulting mixture was allowed to reach ambient temperature (20 ° C) while being stirred and was then heated to 60 ° C. The mixture was then cooled and extracted with methylene chloride (4x100ml). The extracts were combined, washed with water (100ml) and dried using sodium sulphate. The product, 5-tert-butyl-3(2-fluoro-4-chloro-5-methoxyphenyl)-1,3,4-oxadiazol-2(3H)-one was obtained from the extracts by chromatography on a silica column with methylene chloride as eluant. The product was collected from the major fraction eluted from the column as a colourless oil, which subsequently solidified to a white crystalline solid, in a yield of 4.2g, m.pt 89 ° C.

| Analysis: | Calculated: | 51.9% C | 4.7% H | 9.3% N |
|---|---|---|---|---|
| | Found: | 52.2% C | 4.9% H | 9.3% N |

g) The product of f) (3.4g, 0.0113mol) was dissolved in dry methylene chloride and cooled to 10° C. A 1M solution of boron tribromide in methylene chloride (34ml 0.034mol) was added dropwise at a temperature of 10° C under dry nitrogen. After the addition had been completed, the resulting mixture was stirred at ambient temperature (20° C) for 4 hours, carefully quenched with water (250ml) and extracted with methylene chloride. The methylene chloride extract was washed with water and dried using sodium sulphate. Chromatographic purification of the extract on a silica column with trichloromethane as eluant yielded the product. 5-tert-butyl-3-(2-fluoro- 4-chloro-5-hydroxyphenyl)-1,3,4-oxadiazol-2-(3H)-one. The product was collected as the major fraction eluted from the column and obtained as white crystals in a yield of 2.9g, m.pt. 117° C.

| Analysis: | Calculated: | 50.3% C | 4.2% H | 9.8% N |
|---|---|---|---|---|
| | Found: | 50.2% C | 4.4% H | 9.7% N |

Example 2

5-tert-butyl-3-(2-fluoro-4-chloro-5-(1-(ethoxycarbonyl)ethoxy)phenyl)-1.3.4-oxadiazol-2(3H)-one

The product of Example 1, 5-tert-butyl-3-(2-fluoro-4-chloro-5-hydroxyphenyl)-1,3,4-oxadiazol-2(3H)-one, (2.0g, 0.007mol) and anhydrous potassium carbonate (2.0g, 0.014mol) were added to dry acetonitrile (15ml) and the resulting mixture was stirred for 30 minutes. Ethyl 2-bromopropionate (1.8g, 0.01mol) was added and the reaction mixture was heated under reflux with stirring for 27 hours. The solvent was removed. The residue was added to a mixture of 2N hydrochloric acid and methylene chloride and the resulting mixture was stirred. The two phases of the mixture were allowed to separate, the organic phase was removed and the aqueous phase was then extracted with methylene chloride (3x50ml). The extracts were combined with the organic phase, washed with water (50ml) and dried using sodium sulphate. The product, 5-tert-butyl-3-(2-fluoro-4-chloro-5-(1-(ethoxycarbonyl)ethoxy)phenyl)-1,3,4-oxadiazol-2(3H)-one, was separated by chromatography on a silica column eluting with methylene chloride. The product was collected as the major fraction eluted from the column and appeared as a colourless oil which solidified to give a white crystalline solid in a yield of 2.0g, m.pt 75-76° C.

| Analysis: | Calculated: | 52.8% C | 5.2% H | 7.25% N |
|---|---|---|---|---|
| | Found: | 52.9% C | 5.2% H | 7.4% N |

Example 3

5-tert-butyl-3-(2-fluoro-4-chloro-5-ethoxycarbonylmethoxyphenyl)-1,3,4-oxadiazol-2(3H)-one

Using as starting compounds 5-tert-butyl-3-(2-fluoro-4-chloro-5-hydroxyphenyl)-1,3,4-oxadiazol-2(3H)-one (2.0g, 0.007mol) and ethyl bromoacetate (1.7g, 0.01mol, a procedure similar to that described in Example 2 was used to produce the title compound in a yield of 1.4g, m.pt 79-81° C.

| | | | | |
|---|---|---|---|---|
| Analysis: | Calculated: | 51.5% C | 4.8% H | 7.5% N |
| | Found: | 51.8% C | 4.9% H | 7.9% N |

## Example 4

### Herbicidal Activity

To evaluate their herbicidal activity, compounds according to the invention were tested using as a representative range of plants: maize, *Zea mays* (Mz); rice, *Oryza sativa* (R); barnyard grass, *Echinochloa crusgalli* (BG); oat, *Avena sativa* (O); linseed, *Linum usitatissimum* (L); mustard, *Sinapsis alba* (M); sugar beet, *Beta vulgaris* (SB) and soya bean, *Glycine max* (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared from solutions of the test compounds in acetone containing 0.4% by weight of an alkylphenol.ethylene oxide condensate available under the trade mark TRITON X-155. These acetone solutions were diluted with water and the resulting formulations applied at dosage levels corresponding to 5 kg or 1 kg of active material per hectare in a volume equivalent to 600 litres per hectare in the soil spray and foliar spray test, and at a dosage of level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0-9 scale. A rating 0 indicates growth as untreated control, a rating 9 indicates death. An increase of 1 unit on the linear scale approximates to a 10% increase in the level of effect.

The results of the tests are set out in Table I in which the compounds are identified by reference to the preceding examples.

TABLE 1

| Compound of | Soil drench 10/kg/ha | | | | | | | | Dosage | Foliar spray | | | | | | | | Pre-emergence | | | | | | | |
|---|---|---|---|---|---|---|---|---|---|---|---|---|---|---|---|---|---|---|---|---|---|---|---|---|---|
| Ex. No. | Mz | R | BG | O | L | M | SB | S | kg/ha | Mz | R | BG | O | L | M | SB | S | Mz | R | BG | O | L | M | SB | S |
| 2 | 9 | 8 | 9 | 7 | 6 | 9 | 8 | 6 | 5 | 8 | 9 | 9 | 8 | 9 | 9 | 9 | 9 | 9 | 9 | 9 | 7 | 3 | 9 | 9 | 0 |
| | | | | | | | | | 1 | 7 | 9 | 9 | 9 | 9 | 9 | 9 | 9 | 8 | 8 | 9 | 6 | 0 | 9 | 9 | 0 |
| 3 | 8 | 8 | 9 | 7 | 6 | 9 | 8 | 4 | 5 | 8 | 8 | 9 | 8 | 9 | 9 | 9 | 9 | 9 | 8 | 9 | 5 | 0 | 9 | 9 | 0 |
| | | | | | | | | | 1 | 7 | 6 | 9 | 6 | 9 | 9 | 9 | 8 | 8 | 6 | 8 | 3 | 0 | 9 | 9 | 0 |

Example 5

Herbicidal Activity - Temperate species

Further biological evaluations were carried out with compounds according to the invention, which compared their herbicidal properties against temperate species of plants with those of 5-tert-butyl-3-(2,4-dichloro-5-isopropoxyphenyl)-1,3,4-oxadiazol-2(3H)-one, (hereinafter designated as "A") specifically described and exemplified in Example 1 of GB 1,110,500, and 5-tert-butyl-3-(2-fluoro-4-chloro-5-(2-propynoxy)phenyl)-1,3,4-oxadiazol-2(3H)-one, (hereinafter designated as "B") specifically described and exemplified in EP-A-0 274 249.

The tests fall into two categories, pre-emergence and post-emergence. The pre-emergence tests involved spraying liquid formulations of the test compounds onto the soil in which seeds of the plant species mentioned above had recently been sown. The post-emergence tests were foliar spray tests in which seedling plants were sprayed with liquid formulations of the test compounds.

The pre-emergence tests were carried out using as a representative range of temperate species of plants: wheat, Triticum aestivum (WH); barley, Hordeum vulgare (BA); couch grass, Elymus repens (CO); sugar beet, Beta vulgaris (SB); oil seed rape, Brassica napus (RA); speedwell, Veronica persica (SW) and mayweed, Chamomilla spp. (MW).

The post-emergence tests were carried out using as a representatives range of temperature species of plants: wheat, Triticum aestivum (WH), couch grass, Elymus repens (CO); speedwell, Veronica persica - (SW), cleavers, Galium aparine (GG) and field forget-me-not, Myosotis arvensis (FF).

The soil used in the tests was a prepared horticultural loam and was irrigated throughout the tests by means of capillary matting and overhead watering.

The test compounds were formulated in a 1:1 acetone: water mix containing up to 0.2% w/w of an alkylphenol/ethylene oxide wetting agent available under the trade mark, TRITON X-155. The formulations were applied as a single dose in a total volume of 600 litres/hectare. In the pre-emergence tests the test compounds were applied at dosage levels of 3.0 and 1.0 kg/hectare. In the post-emergence tests the test compounds were applied to the plants at dosage levels of 0.3, 0.1, and 0.03 kg/hectare. The dosage levels were selected to produce a range of responses in the plants. At least two replicate pots were used for each treatment with the replicates being distributed in random arrays to minimise the effects of any systematic environmental changes. Untreated seeded soil was used as a control for the pre-emergence tests whilst untreated seedling plants served as controls for the post-emergence tests.

Phytotoxicity compared with the untreated control was assessed visually for each test. The pre-emergence tests were assessed approximately 21 days after treatment and the post-emergence tests were assessed approximately 5 days after treatment. Results were recorded according to a scale of 0 to 9, 0 indicating growth as the untreated control (zero effect) and 9 indicating plant death. An increase of 1 unit on the 0-9 scale approximates to a 10% increase in the level of effect.

The mean recorded result of all the replicates was calculated for each test and converted into a percentage of the total possible effect by multiplying by (100/9). These values are referred to as percentage effect results.

The percentage effect results of the test compounds in the pre-emergence and post-emergence tests are set out in Tables 2 and 3 respectively. The compounds according to the invention are identified by reference to their respective Example numbers.

Table 2

| Pre-Emergence Tests on Temperate Species | | | | | | | | |
|---|---|---|---|---|---|---|---|---|
| Test Compound | Dosage kg/ha | WH | BA | CO | SB | RA | SW | MW |
| Ex. 2 | 3.0 | 0 | 0 | 100 | 100 | 86 | 100 | 100 |
| | 1.0 | 0 | 0 | 68 | 94 | 50 | 94 | 100 |
| Ex. 3 | 3.0 | 39 | 0 | 89 | 100 | 69 | 100 | 100 |
| | 1.0 | 36 | 0 | 80 | 89 | 22 | 94 | 100 |
| A | 3.0 | 44 | 58 | 100 | 100 | 78 | 86 | 100 |
| | 1.0 | 0 | 6 | 81 | 94 | 28 | 64 | 100 |
| B | 3.0 | 36 | 75 | 100 | 100 | 100 | 100 | 100 |
| | 1.0 | 33 | 56 | 97 | 100 | 64 | 97 | 100 |

The results set out in Table 2 indicate that, when applied as a pre-emergence herbicide, the compound of Example 2 exhibits a very high degree of selectivity in both crops of wheat and barley, the compound remaining inactive against wheat and barley at dosages giving a high level of selective control of unwanted species of plants, particularly couch grass, speedwell and mayweed. In addition, the results indicate that the compound of Example 3 remains inactive against barley whilst giving a high level of selective control of unwanted species of plants. In contrast, both prior art compounds A and B have proved to be active against both wheat and barley at dosage levels necessary to effectively control growth of the unwanted plants. It follows, therefore, that neither of the prior art compounds A and B are useful as selective herbicides in crops of wheat or barley.

Table 3

| Post-Emergence tests on Temperate Species | | | | | | |
|---|---|---|---|---|---|---|
| Test Compound | Dosage kg/ha | WH | CO | SW | GG | FF |
| Ex. 2 | 0.3 | 53 | 42 | 86 | 42 | 42 |
| | 0.1 | 25 | 56 | 78 | 28 | 47 |
| | 0.03 | 6 | 0 | 64 | 28 | 36 |
| Ex. 3 | 0.3 | 0 | 0 | 72 | 92 | 72 |
| | 0.1 | 0 | 0 | 61 | 83 | 61 |
| | 0.03 | 0 | 0 | 42 | 81 | 47 |
| A | 0.3 | 28 | 0 | 75 | 83 | 72 |
| | 0.1 | 14 | 0 | 64 | 75 | 61 |
| | 0.03 | 8 | 0 | 50 | 69 | 61 |
| B | 0.3 | 64 | 53 | 86 | 94 | 81 |
| | 0.1 | 58 | 22 | 78 | 83 | 86 |
| | 0.03 | 36 | 6 | 61 | 78 | 64 |

The post-emergence test results set out in Table 3 indicate that the compound of Example 3 is active as a post-emergence selective herbicide in crop of wheat, the compound remaining inactive against wheat but providing control of the growth of unwanted species of plants, particularly speedwell and cleavers. In contrast, both prior art compounds A and B exhibit unacceptable levels of activity against wheat for use as post-emergent selective herbicides in wheat crops.

Example 6

Herbicidal Activity - Tropical Species

Following a general procedure similar to that described in Example 10, additional biological evaluations were carried out with compounds according to the invention, which compared their herbicidal properties against tropical species of plants with those of 5-tert-butyl-3-(2,5-dichloro-5-isopropoxyphenyl)-1,3,4-oxadiazol-2(3H)-one, (hereinafter designated as "A") specifically described and exemplified in GB 1,110,500, and 5-tert-butyl-3-(2-fluoro-4-chloro-5-(2-propynoxy)phenyl-1,3,4-oxadiazol-2(3H)-one, (hereinafter designated as "B") specifically described and exemplified in EP-A-O 274 249.

The pre-emergence tests were conducted using as a representative range of tropical species of plants: soya bean, Glycine max (SO); purslane, Portulaca olevacea (PR); prickly sida, Sida spinosa (PS); jimson weed, Datura stramonium (JW) fat hen, Chenopodium album (FA), and morning glory, Ipomoea purpurea.

The post-emergence tests were conducted using as a representative range of tropical species of plants: soya bean, Glycine max (SO); morning glory, Ipomoea purpurea (MG); purslane, Portulaca olevacea (PR); and barnyard grass, Echinochloa crus-galli (BG).

In the pre-emergence tests the test compounds were applied at dosage levels of 1.92, 0.48 and 0.12 kg/hectare whilst in the post-emergence tests the test compounds were applied at dosage levels of 0.3, 0.1 and 0.03 kg/hectare.

The pre-emergence and post-emergence tests were visually assessed 20 days and 16 days respectively after treatment and the results processed as described in Example 5.

The percentage effect results of the test compounds in the pre-emergence and post-emergence tests are set out in Tables 4 and 5 respectively. The compounds according to the invention are identified by reference to the respective Example numbers.

Table 4

| Pre-emergence tests on tropical species | | | | | | | |
|---|---|---|---|---|---|---|---|
| Test Compound | Dosage kg/ha | SO | PR | PS | JW | FA | MG |
| Ex. 2 | 1.92 | 0 | 100 | 100 | 83 | 100 | 83 |
| | 0.48 | 0 | 100 | 100 | 89 | 97 | 50 |
| | 0.12 | 0 | 69 | 92 | 91 | 83 | 11 |
| Ex. 3 | 1.92 | 0 | 100 | 100 | 97 | 100 | 75 |
| | 0.48 | 0 | 100 | 100 | 69 | 100 | 44 |
| | 0.12 | 0 | 92 | 81 | 0 | 83 | 28 |
| A | 1.92 | 6 | 100 | 100 | 83 | 100 | 78 |
| | 0.48 | 0 | 97 | 94 | 28 | 94 | 53 |
| | 0.12 | 0 | 75 | 81 | 6 | 67 | 28 |
| B | 1.92 | 61 | 100 | 100 | 100 | 100 | 94 |
| | 0.48 | 0 | 100 | 100 | 83 | 100 | 67 |
| | 0.12 | 0 | 100 | 94 | 64 | 97 | 39 |

The results set out in Table 4 indicate that the compounds of both Examples 2 and 3 are inactive against soya at dosage levels necessary to give good control over unwanted plant species such as purslane, jimson weed,fat hen and morning glory. Consequently both compounds exhibit the necessary degree of selectivity for use in controlling unwanted plant growth in crops of soya.

In contrast, whilst inactive against soya at low dosage levels, both prior art compounds A and B exhibit unacceptable levels of activity against soya at the dosage levels necessary to effectively control the growth of such plants as morning glory.

Table 5

| Post-emergence tests on tropical species | | | | | |
|---|---|---|---|---|---|
| Test Compound | Dosage kg.ha | SO | MG | PR | BG |
| Ex. 2 | 0.3 | 17 | 72 | 72 | 61 |
| | 0.1 | 11 | 67 | 56 | 56 |
| | 0.03 | 3 | 75 | 56 | 11 |
| Ex. 3 | 0.3 | 94 | 100 | 100 | 92 |
| | 0.1 | 44 | 100 | 94 | 86 |
| | 0.03 | 17 | 100 | 86 | 56 |
| A | 0.3 | 94 | 100 | 100 | 97 |
| | 0.1 | 53 | 97 | 100 | 64 |
| | 0.03 | 42 | 81 | 86 | 36 |
| B | 0.3 | 100 | 100 | 100 | 100 |
| | 0.1 | 89 | 100 | 100 | 97 |
| | 0.03 | 75 | 100 | 100 | 72 |

The results set out in Table 5 indicate that, whilst at higher dosage levels the compounds of both Examples 2 and 3 are active against soya, lower dosage levels can be employed to which soya is tolerant but which still give a high level of control of such species as morning glory and, in the case of the compound of Example 3, purslane. In contrast, corresponding reductions in the dosage level of the prior art compounds A and B do not sufficiently reduce the level of activity of these compounds against soya. Therefore, unlike the compounds of Examples 2 and 3, the prior art compounds could not be used as selective post-emergent herbicides in crops of soya.

## Claims

1. An oxadiazolone compound having the general formula I:

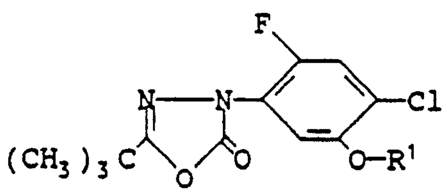

(I)

wherein $R^1$ represents an alkoxycarbonylalkyl group.

2. A compound according to claim 1, wherein $R^1$ represents a ($C_{1-4}$ alkoxy)carbonyl($C_{1-4}$alkyl).

3. A compound according to claim 2, wherein $R^1$ represents a ($C_{1-2}$ alkoxy)carbonyl($C_{1-2}$alkyl).

4. A compound according to claim 3, wherein $R^1$ represents ethoxycarbonylmethyl or 1-(ethoxycarbonyl)ethyl.

5. A process for the preparation of an oxadiazolone compound of formula I as defined in any one of claims 1 to 4, which comprises reacting the compound having formula II:

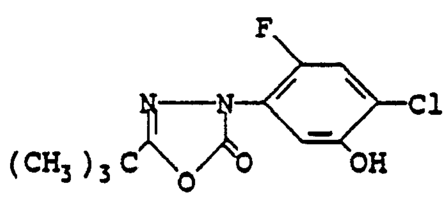

(II)

with a compound of the general formula $R^1$-Y wherein $R^1$ is as defined in any one of claims 1 to 5 and Y represents a leaving group, in a solvent in the presence of a base.

6. A compound of formula I whenever prepared by the process of claim 5.

7. A herbicidal composition comprising a compound of formula I as defined in any one of claims 1 to 4 or claim 6, in association with at least one carrier.

8. The use of a compound according to any one of claims 1 to 4 or claim 6 or of a composition according to claim 7 in combating undesired plant growth.

9. A method of combating undesired plant growth at a locus by treating the locus with a compound of formula I as defined in any one of claims 1 to 4 or claim 6 or a composition as defined in claim 7.

European Patent Office

# EUROPEAN SEARCH REPORT

Application Number

EP 89 20 2058

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | WO-A-8 602 642 (FMC)<br>* Whole document *<br>--- | 1-9 | C 07 D 271/113<br>A 01 N 43/82 |
| D,Y | EP-A-0 274 249 (SHOWA RHODIA CHEM.)<br>* Whole document *<br>--- | 1-9 | |
| Y | FR-A-2 549 841 (SANDOZ)<br>* Whole document, especially, pages 20,21, examples 8-10 *<br>--- | 1-9 | |
| X,Y | GB-A-1 364 137 (RHONE-POULENC)<br>* Whole document *<br>----- | 1-9 | |
| | | | TECHNICAL FIELDS SEARCHED (Int. Cl.5) |
| | | | C 07 D 271/00 |

The present search report has been drawn up for all claims

| Place of search | Date of completion of the search | Examiner |
|---|---|---|
| THE HAGUE | 08-11-1989 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document